# EUROPÄISCHE PATENTSCHRIFT

(11) **EP 1 709 915 B1**
(45) Veröffentlichungstag und Bekanntmachung des Hinweises auf die Patenterteilung: **31.12.2008**
(21) Anmeldenummer: 05077857.0
(22) Anmeldetag: 09.12.2005
(51) Int. Cl.: A61B 17/28, A61B 17/122

(54) **Chirurgische Klemme in der Laparoskopie**
Surgical clip for laparoscopic procedures
Pince chirurgicale laparoscopique

(30) Priorität: 07.04.2005 DE 202005005844 U
(43) Veröffentlichungstag der Anmeldung: 11.10.2006
(73) Patentinhaber: Schnorrenberg Chirurgiemechanik GmbH, 16352 Basdorf (DE)
(72) Erfinder: Schnorrenberg, Arno, 15569 Woltersdorf (DE)
(74) Vertreter: Neumann, Günter

(56) Entgegenhaltungen:
- EP-A- 0 584 723
- WO-A-20/04023976
- US-A- 5 637 110
- US-A1- 2004 172 057

## Beschreibung

Die Erfindung bezieht sich auf eine chirurgische Klemme in der Laparoskopie mit beweglich schließbaren Branchen.

Für die als laparoskopische Operationen bezeichneten Eingriffe im Bauchbereich sind fernbetätigbare über ein Trokar einführbare chirurgische Klemmen, oft auch als Zangen oder Klammern bezeichnet, zum Festhalten und Arretieren von Gewebeteilen, wie Gewebeteilen von Hohlorganen, unverzichtbar.

Eine Anzahl chirurgischer Instrumente, bei denen distale Backen fernbetätigt werden können, um sie zu öffnen und zu schließen, ist bekannt.

Die DE 30 44 186 A1 beschreibt ein Klammerhaltegerät zum Abklemmen von Blutgefäßen. Bei diesem Gerät bildet eine profilierte Federmaterialklinge die beiden Klemmbacken der Klammer. Ein Rohr, in dem die Klammer verschiebbar ist, dient als Schließ- und Öffnungseinrichtung der Klammer.

Aus dem DE 90 10 804.3 U1 geht ein chirurgisches Instrument hervor, das fernbetätigbare Backen eines schwenkbar gelagerten Maulteiles aufweist. Die Backen besitzen jeweils einander zugewandte Aufnahmen für einzusetzende Clips. Für das Festhalten von Gewebeteilen sind sie jedoch nicht geeignet.

Das US-Patent Nr. 4,064,881 offenbart eine chirurgische Federklemme, die aus beweglichen Klauen mit einer griffigen inneren Oberfläche besteht und die ergänzende Zähne und Vertiefungen aufweisen.

Die US-Patente Nr. 5,776,146 und 5,776,147 beschreiben chirurgische Klammern mit zwei gegenüberliegenden Klemmbacken, von denen eine Klemmbacke relativ zu der zweiten Klemmbacke gelagert ist, deren Innenfläche mit quer zur Längsachse verlaufenden Rillen versehen ist. Die beweglich gelagerte Klemmbacke weist an ihrem Ende einen annähernd rechtwinklig angeordneten Haken auf, der im geschlossenen Zustand über das distale Ende der zweiten Klemmbacke greift und so ein Festhalten eines Darmes oder anderen Gewebeteiles sichern soll. Eine spezielle Ausführungsform sieht vor, dass der Haken in die Ausnehmung eines Vorsprunges greift, der am distalen Ende der unbeweglichen Klemmbacke angeordnet ist.

Das US-Patent 5,637,110 offenbart eine Greifvorrichtung deren Branchen auf den Innenseiten sägezahnartig ausgebildete und quer zur Längsachse der Branchen verlaufende verhältnismäßig scharfkantige Rippen aufweisen. Bei dieser konstruktiven Ausbildung der Rippen sind Gewebeverletzungen verbunden mit Blutungen nicht auszuschließen.
Die europäische Patentanmeldung EP 0 584 723 A1 beschreibt ebenfalls eine Greifvorrichtung mit Branchen, deren quer zur deren Längsachse verlaufenden Rippen beim Schließen der Branchen polygonale Öffnungen bilden. Das wulstartig ausgebildete distale Ende der Branchen steht jedoch einem optimalen Handling der Organe im Wege.
Die mit der WO 2004/023976 A2 offenbarte Greifvorrichtung weist Branchen auf, die auf ihren Innseiten mit Abstand voneinander aufgesetzte Rippen aufweisen. Das distale Ende der Greifvorrichtung ist wulstartig und stufenförmig zu einer Art Trichter ausgebildet. Ein optimales Handling der Organe ist mit dieser Greifvorrichtung nicht gegeben.

Aus den US-Patenten Nr. 5,366,477, 5,590,570, 5,695,522 und 5,727,428 geht eine chirurgische Greifvorrichtung hervor, die eine stationäre untere Backe und eine beweglich gelagerte obere Backe aufweist. Die Oberflächen der Backen sind auf der jeweils zugewandten Seite mit quer zur Längsachse der Backen wellenförmig ausgebildeten Ausnehmungen versehen. Im geschlossenen Zustand der Greifvorrichtung bilden deren Backen einen vom distalen zum basalen Ende der Backen verlaufenden breiter werdenden Spalt. Eine derartige Lösung offenbart auch die US 2004/0172057 A1.

Die genannten Klemmen, Zangen und Greifvorrichtungen haben sämtlich den Nachteil, dass sie beim Einsatz an Hohlorganen, wie Dickdarm, Dünndarm und Magen, ein zuverlässiges Festhalten von Gewebeteilen nicht gewährleisten. Die meist punktuellen Ansatzflächen führen beim Greifen der Organe zu punktförmigen Belastungen mit der Gefahr von Gewebseinreißungen mit Blutungen oder partiellen bis kompletten Wandzerstörungen von Hohlorganen. Die überwiegend scharfkantigen oder spitz verlaufenden oder mit scharfkantigen Zähnen verlaufenden Branchen erhöhen dieses Risiko noch. Die überwiegend minimierten Greifflächen bedingen darüber hinaus eine Gefährdung durch Zug am Gewebe und sind insbesondere problematisch bei voluminösem Gewebe.

Infolge von Adipositas ist das gegenwärtig bei ca. 50 % der Patienten gegeben. Dieser Umstand lässt gerade die adipösen Patienten von laparoskopischen Untersuchungsmethoden ausschließen.

Aufgabe der Erfindung ist es daher, eine verbesserte chirurgische Klemme für laparoskopische Operationen bereitzustellen, die verletzungsfrei geklemmtes Gewebe zuverlässig arretiert.

Erfindungsgemäß wird die Aufgabe durch eine chirurgische Klemme mit den Merkmalen des Anspruches 1 gelöst. Vorteilhafte Ausgestaltungen der erfindungsgemäßen Klemme ergeben sich aus den Merkmalen der Ansprüche 2 bis 4.

Mit der Erfindung verbindet sich der besondere Vorzug, dass eine Klemme mit einer ideal atraumatischen Greiffläche bereitgestellt werden kann. Das geklemmte Gewebe wird durch die Greifflächen mit den quer zur Längsachse verlaufenden Ausnehmungen und stumpf geformten Rippen und durch die in die Branchenöffnungen gequetschten Gewebeanteile optimal arretiert. Die erfinderische Ausgestaltung der Branchen verhindert ein Abgleiten des gefassten Gewebes mit unbemerkten Ausreißungen. Das abgerundete distale Ende der Klemme verhindert eine tangentiale Gewebstraumatisierung und ermöglicht zugleich die stumpfe Präparation des Gewebes ohne Gewebszerschneidung oder Einreißung. Sie gewährleistet eine breite Gewebserfassung sowohl am Hohlorgan als auch im fettreichen Gekröse.

Im Folgenden soll die chirurgische Klemme in der Laparoskopie an Hand von Zeichnungen näher erläutert werden. Es zeigen:
- **Fig. 1**: die Seitenansicht auf eine Ausführungsform der chirurgischen Klemme mit geöffneten Branchen
- **Fig. 2**: die Seitenansicht der Ausführungsform der chirurgischen Klemme gemäß Fig. 1 im geschlossenen Zustand

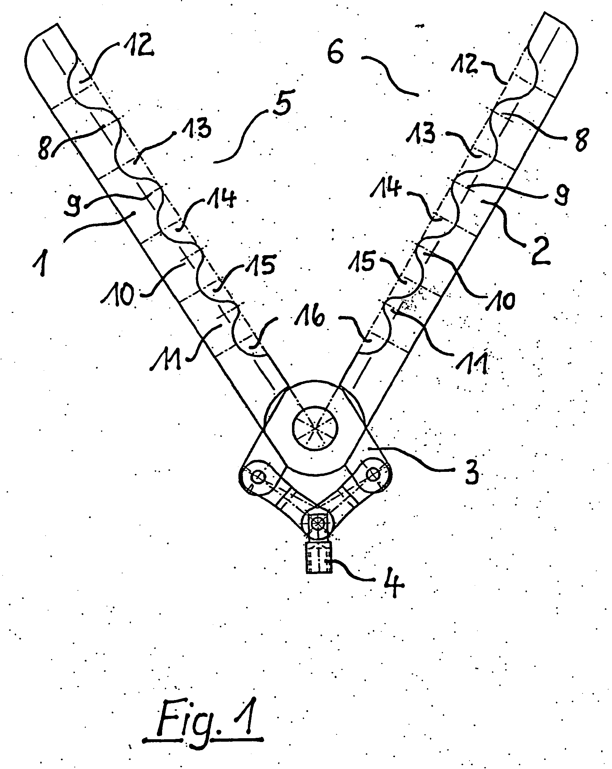

Die in **Fig. 1** dargestellte Ausführungsform der Erfindung zeigt in Seitenansicht die chirurgische Klemme im geöffneten Zustand mit den Branchen 1 und 2, die hier beide beweglich gelagert sind. Über ein Scherengelenk 3 können beide Branchen 1 und 2 durch Betätigen der Zugstange 4 geöffnet oder geschlossen werden.
Wie aus Fig. 1 zu erkennen, besitzen die Branchen 1 und 2 die Greifflächen 5 und 6, die quer zu den Längsachsen der Branchen 1 und 2 verlaufende Ausnehmungen 12, 13, 14, 15 und 16 und abgerundet geformte Rippen 8, 9, 10 und 11 aufweisen.

Die Erfindung kann auch mit einer stationären Branche und einer beweglich gelagerten Branche ausgeführt werden. In Verbindung mit einem an sich bekannten Gelenk, beispielsweise einem Kniehebelgelenk, kann die bewegliche Branche mittels einer Zugstange betätigt und auf diese Weise die Klemme ebenfalls geöffnet und geschlossen werden.

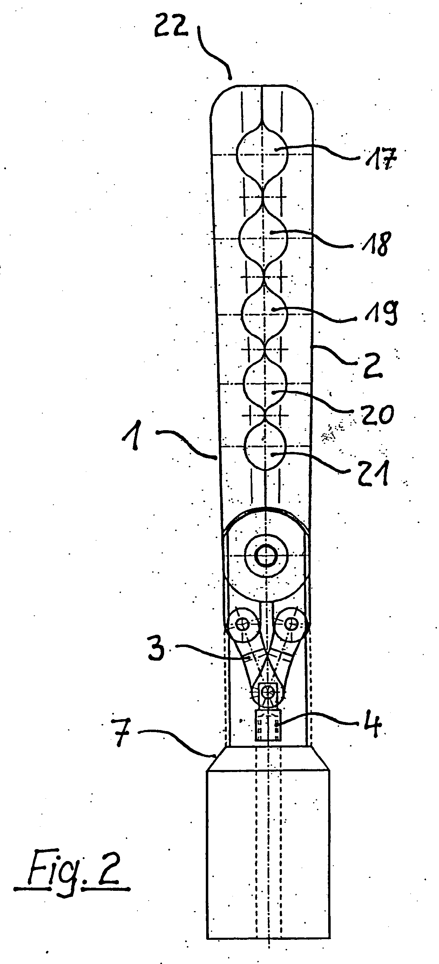

**Fig. 2** zeigt in Seitenansicht die Ausführungsform der in **Fig. 1** dargestellten Klemme im geschlossenen Zustand, geführt in dem Hüllrohr 7.
Deutlich ist zu erkennen, dass die Branchen 1 und 2 gleichartig geformte Greifflächen 5 und 6 aufweisen. Die Rippen 8, 9, 10 und 11 der Greifflächen 5 und 6 sind abgerundet geformt und stumpf ausgeführt und stoßen im geschlossenen Zustand der Branchen 1 und 2 aneinander, so dass die Ausnehmungen 12, 13, 14, 15 und 16 bei dieser Ausführung der erfindungsgemäßen Klemme die fünf Branchenöffnungen 17, 18, 19, 20 und 21 mit einem annähernd kreisförmigen Querschnitt bilden. Die Branchenöffnungen 17 bis 21 können auch eine andere Geometrie des Querschnittes aufweisen, wie beispielsweise den einer Ellipse.
Das distale Ende 22 der Klemme mit einer Breite von ca. 10 mm ist fingerförmig abgerundet und ebenfalls stumpf ausgeführt. Basal hat sich eine Breite der Klemme von 7 mm als vorteilhaft erwiesen. Mit einem Durchmesser von max. 10 mm und bei einer Länge der Greifflächen 5 und 6 von ca. 35 mm ist die Klemme universell und zuverlässig einsetzbar, so in der laparoskopischen Kolorektalchirurgie, bei laparoskopischen Magenresektionen, der laparoskopischen Splenektomie, bei laparoskopischen Teilresektionen der Leber, in der laparoskopischen Cholecystektomie bei akuter Galle und in der laparoskopischen bariatischen Chirurgie.

Die für die laparoskopische Abdominalchirurgie entwickelte Klemme eignet sich besonders zur Führung am Kolorektum und Magen. Die erfindungsgemäße Klemme kann über alle Portale ab 10 mm eingebracht werden. Durch das Fehlen einer Sperre wird das Instrument mittels digitaler Führung eingesetzt, wodurch der Gewebsandruck individuell zum Einsatz kommt. Die Einführung und die Neuplatzierung der Klemme erfolgt mit geschlossenen Branchen 1 und 2, um eine unbewusste Gewebsverletzung zu vermeiden. Der Einsatz am Darm oder Gekröse kann sowohl tangential als auch frontal erfolgen. Vorteilhaft ist es, die Klemme unter Kamerasicht einzusetzen. Das ist insbesondere beim Einsatz an Organen geboten.

In einer Vortestung ist die erfindungsgemäße Klemme bei 360 Operationen am Kolorektum mit Hemikolektomie rechts, Transversumresektionen, Hemikolektomie links, Sigmaresektionen und Rektumresektionen universell eingesetzt worden. Es wurde keine Verletzung am Gekröse oder Hohlorgan durch die Klemme beobacht. Gleiche Erfahrungen sind mit der Klemme in der Magenchirurgie mit Resektionen und bei der paraösophagealen Hernie gesammelt worden. Als überzeugend haben sich auch die Einsätze bei der Splenektomie mit atraumatischer Führung der Milz bei der Skelettierung erwiesen. Entzündlich veränderte und wandverdickte Gallenblasen sind mit der erfindungsgemäßen Klemme optimal zu handeln, Instrumentenperforationen, wie sie mit anderen Fasszangen nicht selten auftreten, wurden nicht festgestellt.

Es ist bekannt, dass im laparoskopischen Einsatz durch die Kameraorientierung auf die aktuelle Gewebsdissektion die Halteklemme außerhalb des Gesichtsfeldes liegt. Daher stellen Gewebsdruck und -zug, überwiegend durch die linke Hand ausgeübt, ein außerordentliches Gefahrenmoment dar.
Die erfindungsgemäße laparoskopische Klemme ist dagegen sicher im Griff und sicher in der Vermeidung von Gewebszerreißungen. Unbemerkte Blutungen und Hohlorganeröffnungen durch das Instrument sind zuverlässig vermeidbar. Die Führung des Instruments durch das Abdomen, auch außerhalb des Gesichtsfeldes des Operateurs, gestaltet sich durch die stumpfe fingerförmige Gestaltung des distalen Endes 22 der Klemme gefahrlos. Die Klemme ist zum Fassen von Hohlorganen und Gekröse bestens geeignet.

Die Präparation mit dem distalen Ende 22 der Klemme sollte jedoch nicht außerhalb von anatomischen Gewebsspalten vollzogen werden. Die Sicherung von isolierten Blutgefäßen ist durch die Beschaffenheit der Branchen (1; 2) jedoch nicht möglich. Ebenso ist die Klemme nicht zum Führen von Nadeln einsetzbar.

Ungeachtet des bei jedem chirurgischen Instrument begrenzten Einsatzgebietes erweist sich die erfindungsgemäße Klemme als eine chirurgische Universalklemme in der Laparoskopie.

### BEZUGSZEICHENAUFSTELLUNG

- 1: Branche
- 2: Branche
- 3: Scherengittergelenk
- 4: Zugstange
- 5: Greiffläche
- 6: Greiffläche
- 7: Hüllrohr
- 8: Rippen
- 9: Rippen
- 10: Rippen
- 11: Rippen
- 12: Ausnehmung
- 13: Ausnehmung
- 14: Ausnehmung
- 15: Ausnehmung
- 16: Ausnehmung
- 17: Branchenöffnung
- 18: Branchenöffnung
- 19: Branchenöffnung
- 20: Branchenöffnung
- 21: Branchenöffnung
- 22: distales Ende

## Patentansprüche

1. Chirurgische Klemme in der Laparoskopie mit zwei Branchen (1; 2) von denen mindestens eine Branche beweglich gelagert ist und deren Greifflächen (5; 6) jeweils mindestens zwei quer zur Längsachse der Branchen (1; 2) verlaufende und durch Rippen (8; 9; 10; 11) voneinander getrennte Ausnehmungen (12; 13; 14; 15; 16) aufweisen, die Rippen (8; 9; 10; 11) im geschlossenen Zustand der Branchen (1; 2) aufeinanderstoßen und die Ausnehmungen (12; 13; 14; 15; 16) mindestens zwei Branchenöffnungen (17; 18; 19; 20; 21) bilden, **dadurch gekennzeichnet, dass** die Branchen (1; 2) in ihrer Innenseite wellenförmig ausgebildet sind und die Innenseiten am distalen Ende jeweils einen planen Abschnitt aufweisen, die Branchenöffnungen (17; 18; 19; 20; 21) einen annähernd kreisförmigen oder ellipsenförmigen Querschnitt besitzen und die durch die Branchenöffnungen (17; 18; 19; 20; 21) im Querschnitt gebildeten Flächen in ihrer Größe vom distalen zum basalen Ende der Branchen (1; 2) abnehmen.

2. Chirurgische Klemme nach Anspruch 1, **dadurch gekennzeichnet, dass** die Klemme im geschlossenen Zustand der Branchen (1; 2) fünf Branchenöffnungen (17; 18; 19; 20; 21) aufweist.

3. Chirurgische Klemme nach Anspruch 1 oder 2, **dadurch gekennzeichnet, dass** die Klemme am distalen Ende (22) der Branchen (1; 2) eine größere Höhe als am basalen Ende aufweist und sich diese Höhe zum basalen Ende hin gleichmäßig verringert.

4. Chirurgische Klemme nach einem der Ansprüche 1 bis 3, **dadurch gekennzeichnet, dass** die Rippen (8; 9; 10; 11) der Greifflächen (5; 6) und Kanten der Branchen (1; 2) abgerundet ausgeführt sind.

5. Chirurgische Klemme nach einem der Ansprüche 1 bis 4, **dadurch gekennzeichnet, dass** die Klemme im geschlossenen Zustand ein fingerförmig gerundetes distales Ende (22) aufweist.

## Claims

1. Surgical clamp in laparoscopy with two branches (1; 2) of which at least one branch is moveable and whose gripping surfaces (5; 6) display at least two separate hollows (12; 13; 14; 15; 16) with ribbing (8; 9; 10; 11) running perpendicular to the length of the branches (1; 2), the ribs (8; 9; 10; 11) collide with one another when the branches (1; 2) are closed and the hollows (12; 13; 14; 15; 16) form at least two branch openings (17; 18; 19; 20; 21), **characterized by** the branches (1; 2) being wave-shaped on the inside and the insides having a smooth section on the distal end, the branch openings (17; 18; 19; 20; 21) having an almost circular or elliptical cross-section and the areas formed by the cross-section of the branch openings (17; 18; 19; 20; 21) decrease in size from the distal to the basal end of the branches (1; 2).

2. Surgical clamp in accordance with claim 1, **characterized by** the clamp having five branch openings (17; 18; 19; 20; 21) when the branches are closed (1; 2).

3. Surgical clamp in accordance with claim 1 or 2, **characterized by** the clamp on the distal end (22) of the branches (1; 2) having a higher end than the basal end and that this height decreases consistently to the basal end

4. Surgical clamp in accordance with claims 1 to 3, **characterized by** the ribs (8; 9; 10; 11) of the gripping surfaces (5; 6) and the edges of the branches (1; 2) being rounded.

5. Surgical clamp in accordance with one of the claims 1 to 4, **characterized by** the clamp displaying a finger-shaped rounded distal end (22) when closed.

## Revendications

1. Pince chirurgicale de laparoscopie à deux branches (1; 2), dont au moins une est articulée sur paliers et dont les surfaces de préhension (5 ;6) présentent respectivement au moins deux évidements (12; 13; 14; 15; 16), perpendiculaires à l'axe longitudinale des branches (1; 2) et séparés par des nervures (8; 9; 10; 11). Lorsque les branches de la pince (1; 2) sont refermées, les nervures (8; 9; 10; 11) butent les unes contre les autres et les évidements (12; 13; 14; 15; 16) forment au moins deux ouvertures (17; 18; 19; 20; 21) de branches. La pince se **caractérise par le fait que** la face intérieure des branches (1; 2) est ondulée et que les faces intérieures à l'extrémité distale présentent respectivement une section plane. Les ouvertures de branches (17; 18; 19; 20; 21) présentent une coupe transversale presque ronde ou elliptique. La taille des surfaces formées par les ouvertures de branches (17; 18; 19; 20; 21) dans la coupe transversale, diminue de l'extrémité distale à l'extrémité basale des branches (1; 2).

2. Pince chirurgicale conforme aux spécifications 1, **caractérisée par le fait que,** lorsque ses branches (1; 2) sont fermées, la pince présente cinq ouvertures de branches (17; 18; 19; 20; 21).

3. Pince chirurgicale conforme aux spécifications 1 ou 2, **caractérisée par le fait que** la pince présente une hauteur plus importante au niveau de l'extrémité distale (22) des branches (1; 2) qu'au niveau de l'extrémité basale, et que cette hauteur diminue de façon régulière vers l'extrémité basale.

4. Pince chirurgicale conforme à l'une des spécifications 1 à 3, **caractérisée par le fait que** les nervures (8; 9; 10; 11) des surfaces de préhension (5; 6) et les arêtes des branches (1; 2) sont arrondies.

5. Pince chirurgicale conforme à l'une des spécifications 1 à 4, **caractérisée par le fait que** la pince fermée présente une extrémité distale (22) arrondie digitiforme.
